# EUROPEAN PATENT APPLICATION

(11) **EP 4 464 768 A1**
(43) Date of publication of application: **20.11.2024**
(21) Application number: 24176578.3
(22) Date of filing: 17.05.2024
(51) Int. Cl.: C12M 1/00

(54) **LIGHT DIFFUSER**

(30) Priority: 19.05.2023 FI 20235562
(71) Applicant: Agri-Biotech Oy, 65760 Iskmo (FI)
(72) Inventor: Heiskanen, Harri, 65760 Iskmo (FI); Jaatinen, Pekka, 65760 Iskmo (FI)
(74) Representative: Kolster Oy Ab

(57) **Abstract**

The invention relates to a fluid tight light diffuser (1), the light diffuser (1) comprising an elongated diffuser body (10) comprising a first end (10a), and a second end (10b), the first end (10a) receiving a light from a light source (3). Further, the diffuser body (10) of a first material contains irregularities in said first material providing diffusing structures (11; 11'; 11"; 11‴) for diffusing the light of the light source (3), such that the light exits the diffuser body (10) at a predefined intensity in different parts over the length of the diffuser body (10).

## Description

### BACKGROUND OF THE INVENTION

### FIELD OF THE INVENTION

This invention relates to a light diffuser, particularly to a light diffuser suitable for underwater illumination in algae cultivation.

### DESCRIPTION OF PRIOR ART

As photosynthetic organisms, microalgae require light, CO2, appropriate temperature (generally 15-35°C), appropriate pH (1.5-10, but generally 6-7.5), and a supply of nutrients. Some algae may also consume organic carbon when it is available. However, light and CO2 are the two most important growth factors for phototrophic microalgae. Light provides the energy for microalgal biomass production, whereas CO2 provides the carbon for that biomass. Provision of light is largely dependent on the reactor design, which may be an open system, such as a pond, or a closed photobioreactor (PBR). In either case, light may be obtained directly from sunlight, or with artificial light.

Light is necessary for microalgal photosynthesis, yet excessive or insufficient incident light constrains optimal performance of the microalgae cultivation in terms of biomass or metabolite yields. Particularly, also the light intensity, frequency, and spectrum affect the performance of the cultivation.

One major problem in designing efficient bioreactors for phototrophic microalgae arises from the fact that light attenuates exponentially as it penetrates into the culture medium. The typical light requirement is about 100 µmol m-2 s-1 but can go up to 2000 µmol m-2 s-1 depending on the product being produced. Therefore, unless the culture exhibits a high ratio of area per volume of the culture vessel, or a low cell density, sufficient light will be available only in a narrow zone close to the culture surface.

In conventional algae cultivation methods, the artificial light source is placed above the surface of the cultivation medium or placed next to the pool/tank in a lateral direction.

In order to overcome the short light penetration depth, in known solutions, the distance issue between the innermost region of the culture and the light source has been attempted to be solved with either very shallow structure of the cultivation tank, or strong movement of the cultivation medium. However, both of these solutions have significant drawbacks such as energy efficiency, space efficiency, or CO2 availability.

Therefore, there exists a clear need for an improved way of providing sufficient light conditions for microalgae cultivation.

### SUMMARY OF THE INVENTION

An object of the present invention is to solve the above-mentioned disadvantages and to provide a light diffuser for algae cultivation which has excellent use properties for algae cultivation and can be manufactured reliably. These and other objects are achieved with a light diffuser according to independent claim 1.

Preferred embodiments of the invention are disclosed in the dependent claims.

Further advantages and details of the invention are disclosed in detail in the description below.

### BRIEF DESCRIPTION OF DRAWINGS

In the following the present invention will be described in closer detail by way of example and with reference to the attached drawings, in which
figure 1 illustrates a perspective view of a first embodiment of the invention,
figure 2 illustrates a perspective view of a second embodiment of the invention,
figure 3 illustrates a perspective view of a third embodiment of the invention,
figure 4 illustrates a perspective view of a fourth embodiment of the invention, and
figure 5 illustrates an assembly comprising the second embodiment of the invention.

### DESCRIPTION OF AT LEAST ONE EMBODIMENT

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned. All combinations of the embodiments are considered possible if their combination does not lead to structural or logical contradiction.

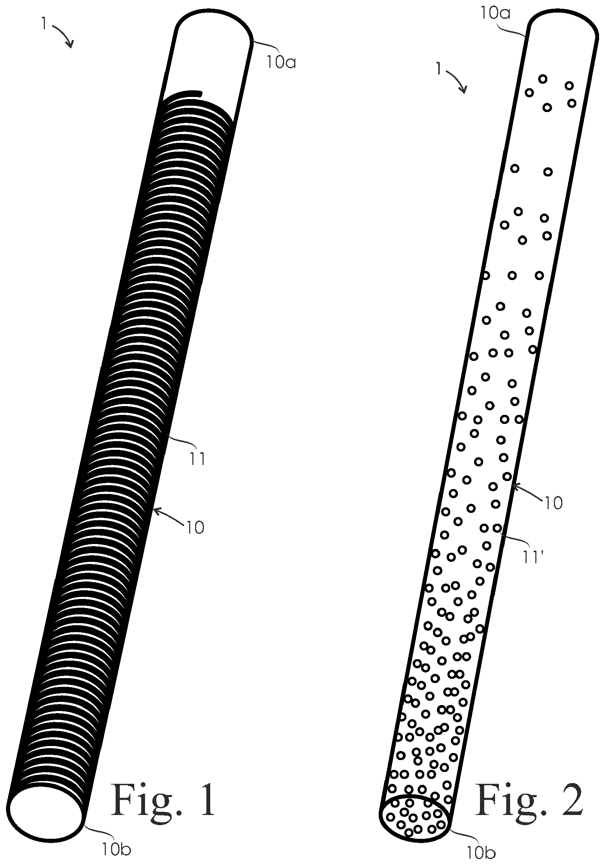

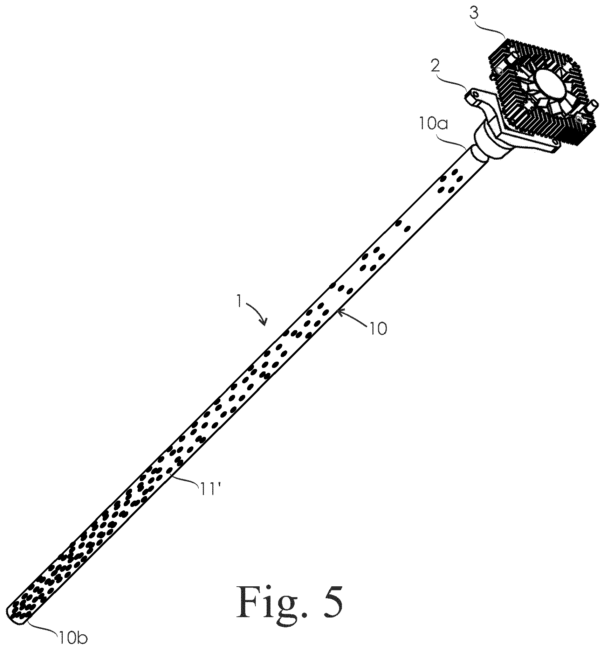

Figures 1 to 4 illustrate embodiments of a fluid tight light diffuser 1. The light diffuser 1 comprises an elongated diffuser body 10 comprising a first end 10a, and a second end 10b. The first end 10a receiving a light from a light source 3 as illustrated in the assembly of figure 5.

The diffuser body 10 of a first material contains irregularities in said first material providing diffusing structures 11; 11'; 11"; 11‴ for diffusing the light of the light source 3, such that the light exits the diffuser body 10 at a predefined intensity in different parts over the length of the diffuser body 10. The irregularities in said first material cause the light to diffuse and thus affect the intensity of the light emitted by the light diffuser. The irregularities may be formed in several ways, some embodiments of the irregularities are shown in figures 1 to 4 as the diffusing structures 11; 11'; 11"; 11‴. The invention allows the light diffuser to be immersed in the cultivation medium in an algae tank, thus allowing the algae to be illuminated directly in the cultivation tank. This improves the energy efficiency of algae cultivation and the irregularities providing the diffusing structures 11; 11'; 11"; 11‴ allow the light intensity to be optimized for the environment of use.

Preferably, the light diffuser 1 is a light diffuser 1 for underwater illumination in algae cultivation.

Preferably, the light diffuser 1 further comprises a transparent sleeve surrounding the diffuser body 10, the transparent sleeve having a smooth outer surface. Depending on the type of diffusing structures 11; 11'; 11"; 11‴ formed in the diffuser body 10, the outer surface of the diffuser body 10 may be susceptible to induce unwanted algae growth on the light diffuser 1 which decreases the illumination effect and requires regular cleaning. Thus, the transparent sleeve provides an outer surface for the light diffuser 1, which is less prone to algae growth on the surface, and is easier to clean without weaking the light emitting properties of the light diffuser 1. Using a sleeve also facilitates a quicker cleaning process, as the sleeve may simply be replaced with a new clean one. This reduces the downtime of the illumination and increases efficiency of use. Particularly, the embodiments of figures 1, 3 and 4 benefit from a transparent sleeve due to contours of the outer surface of the diffuser body 10 in said embodiments, however the sleeve may be used in combination with any embodiment. The sleeve 20 is preferably also cylindrical and fits closely on the diffuser body 10.

In an embodiment, the predefined intensity in different parts varies between the different parts of the diffuser body 10. The intensity may be varied by varying the irregularities in the diffuser body 10 providing the light diffusing structures 11; 11'; 11"; 11‴. This is beneficial as it further allows the intensity provided by the light diffuser 1 to be controlled more precisely. One beneficial use case is when an algae tank is also exposed to other outside lighting, for instance natural light. In such case, it may be beneficial to decrease the light intensity near the top of the algae tank in comparison to the bottom of the algae tank.

In an embodiment, the predefined intensity in different parts is uniform over the length of the diffuser body 10. This allows the algae to be illuminated evenly over the length of the diffuser body 10 without having areas illuminated with too high intensity that could cause photoinhibition, photo-oxidation, and the algae to die, or with too low intensity which would not allow for proper photosynthesis.

Fluid tightness of the light diffuser 1 decreases issues which could result from water and algae entering the light diffuser. In the embodiment of figures 1 to 4, the diffuser body 10 is a solid cylindrical rod, making it a fluid tight structure in itself. Alternatively, the diffuser body 10 could be tubular, in which case, depending on the diffusing structures 11; 11'; 11"; 11‴ of the light diffuser 1, either the diffuser body 10 is fluid tight without openings, or the transparent sleeve 20 is required to provide the fluid tightness in case of through holes, for instance.

Figure 1 illustrates a first embodiment of the light diffuser 1. In the first embodiment the light diffusing structures 11 are formed as lateral grooves in the diffuser body 10. The lateral grooves may be formed on the diffuser body 10 directly when forming the diffuser body 10 or by removing material from the diffuser body 10 by for example machining. The simple geometry of the grooves allows the diffusing structures 11 to be cost-effectively formed on the diffuser body 1.

In an embodiment not illustrated, the grooves increase in width from the first end 10a of the diffuser body 10 to the second end 10b of the diffuser body 10. In an embodiment not illustrated, the grooves increase in depth from the first end 10a of the diffuser body 10 to the second end 10b of the diffuser body 10. In an embodiment not illustrated, the grooves increase in frequency from the first end 10a of the diffuser body 10 to the second end 10b of the diffuser body 10. These solutions increase the surface area of the diffuser body 10 covered by the diffusing structures 11 from first end 10a of the diffuser body 10 to the second end 10b of the diffuser body 10, thus affecting the light diffusion properties of the light diffuser 1. Also, a combination of the above may be used to achieve the desired light diffusing profile of the light diffuser 1.

Figure 2 illustrates a second embodiment of the light diffuser 1. In the second embodiment, the light diffusing structures 11' are formed as bubbles in the diffuser body 10. The bubbles may be formed in the diffuser body 10 during manufacturing of the diffuser body.

In an embodiment not illustrated, the bubbles increase in diameter from the first end 10a of the diffuser body 10 to the second end 10b of the diffuser body 10. In the embodiment of figure 2, the frequency of the bubbles increases from the first end 10a of the diffuser body 10 to the second end 10b of the diffuser body 10. These solutions increase the surface area of the diffuser body 10 covered by the diffusing structures 11' from first end 10a of the diffuser body 10 to the second end 10b of the diffuser body 10, thus affecting the light diffusion properties of the light diffuser 1. Also, a combination of the above may be used to achieve the desired light diffusing profile of the light diffuser 1.

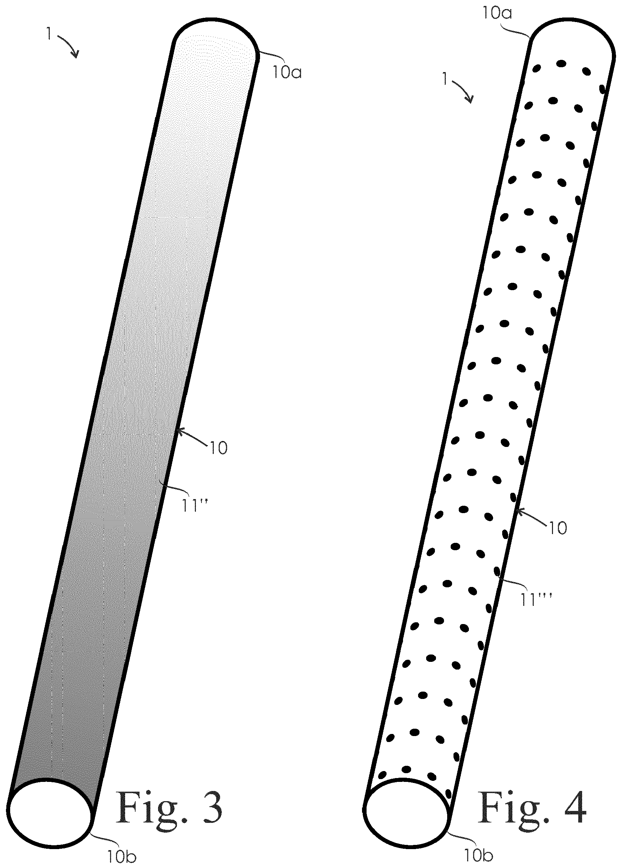

Figure 3 illustrates a third embodiment of the light diffuser 1. In the third embodiment, the light diffusing structures 11" are formed as surface roughness on the diffuser body. This surface roughness can be formed on the diffuser body by roughening it with a roughening machine. In the third embodiment, the surface roughness increases from the first end 10a of the diffuser body 10 to the second end 10b of the diffuser body 10, as illustrated with the gradient in figure 3. This increases the diffusing properties of the light diffuser 11" from first end 10a of the diffuser body 10 to the second end 10b of the diffuser body 10, thus affecting the light diffusion properties of the light diffuser 1.

Figure 4 illustrates a fourth embodiment of the light diffuser 1. In the fourth embodiment, the light diffusing structures 11‴ are formed as radial holes on the diffuser body 10. The holes preferably are through holes that extend through the diffuser body 10. The holes can easily be drilled in the diffuser body 10, after the diffuser body has been formed.

In an embodiment not illustrated, the radial holes increase in diameter from the first end 10a of the diffuser body 10 to the second end 10b of the diffuser body 10. In an embodiment not illustrated, the radial holes increase in frequency from the first end 10a of the diffuser body 10 to the second end 10b of the diffuser body 10. In an embodiment not illustrated, the radial holes increase in depth from the first end 10a of the diffuser body 10 to the second end 10b of the diffuser body. These solutions increase the surface area of the diffuser body 10 covered by the diffusing structures 11‴ from first end 10a of the diffuser body 10 to the second end 10b of the diffuser body 10, thus affecting the light diffusion properties of the light diffuser 1 Also, a combination of the above may be used to achieve the desired light diffusing profile of the light diffuser 1.

In an embodiment not illustrated, the diffuser body 10 further comprises fillers in at least some of the radial holes. The fillers may be clear or colored in order to further adjust either the diffusion and/or the wavelength of the light. Different species of algae or other plants thrive at different wavelengths or intensities of light or enhance desired qualities in the plants. This allows the same light source 3 to be used for different purposes by simply changing the light diffuser 1 to one having a suitable configuration.

The colored fillers may be of any suitable color in the visible spectrum, such as red, blue, or green for instance. Fillers of different color absorb different wavelengths of light and allow others to pass. This in turn affects the light transmitted by the light diffuser without the need to replace the light source 3.

In an embodiment not illustrated, the color of the fillers varies depending on the region of the diffuser body 10 in which each filler is located. For example, the fillers nearest to the first end 10a of the diffuser body 10 may be transparent, in a next region towards the second end 10b of the diffuser body 10 the fillers may be red, and the fillers nearest to the second end 10b of the diffuser body 10 may be green. With various configurations, different aspects of the light can be prioritized for different species or different parts of the plant i.e the stem and the leaves can be provided with light of different wavelength. Also, different species or plants at different growth stages may be simultaneously illuminated at optimized wavelengths by use of the same light diffuser 1.

So, in other words the color of the fillers located near first end 10a of the diffuser body 10 may differ from the color of the fillers located near second end 10b of the diffuser body 10 to vary the transmitted wavelength of the light over the length of the diffuser body 10.

Preferably, the fillers are polycarbonate or acrylic such as polymethylmethacrylate (PMMA). However, any suitable light conducting material may be used.

The fillers may be either detachably attached in the radial holes or integrally formed in the diffuser body 10 during manufacturing. Detachable attachment allows the fillers to be changed and same diffuser body 10 to be utilized with different configurations. This is particularly advantageous when optimal growth conditions are tested, and the growth process is optimized. Integrally formed fillers do not allow the filler configuration to be altered but provide a more stable structure for use conditions where the light diffuser 1 may experience outside forces caused by the environment.

In the embodiments of figures 1 to 4, the elongated diffuser body 10 is cylindrical. This shape is beneficial as the round cross-section does not comprise sharp corners, and therefore facilitates even distribution of the light.

In an embodiment, the diffuser body 10 is tubular, and comprises a uniform wall thickness surrounding a cavity from first end 10a of the diffuser body 10 to the second end 10b of the diffuser body 10. Preferably, the first end 10a of the diffuser body 10 is then open and the second end 10b of the diffuser body is closed.

Preferably, the first material of the diffuser body 10 comprises polymethylmethacrylate (PMMA).

Preferably, the transparent sleeve comprises polymethylmethacrylate (PMMA).

Figure 5 illustrates an assembly comprising the third embodiment of the invention illustrated in figure 3. The assembly further comprises a light source 3 and a connecting element 2 for connecting the light diffuser 1 to the light source 3, such that the first end 10a of the diffuser body 10 is inserted into the connecting element 2.

This assembly is intended to be used in an algae tank, such that the light diffuser 1 is immersed in the cultivation medium of the algae tank, and the light source remains outside of the cultivation medium. This way the light can be introduced directly to the cultivation medium, and effects of the short light penetration depth are minimized and a more energy efficient lighting is achieved. Thus, the assembly facilitates higher yields in algae cultivation and improves energy efficiency of the cultivation. Depending on the scale of the tank, a plurality of said assemblies may be used in the tank to provide the entire volume of the tank with optimal lighting conditions.

It is to be understood that the above description and the accompanying figures are only intended to illustrate the present invention. It will be obvious to a person skilled in the art that the invention can be varied and modified without departing from the scope of the invention.

## Claims

1. A fluid tight light diffuser (1) for underwater illumination in algae cultivation, the light diffuser (1) comprising:
an elongated diffuser body (10) comprising a first end (10a), and a second end (10b), the first end (10a) receiving a light from a light source (3), wherein
the diffuser body (10) of a first material contains irregularities in said first material providing diffusing structures (11") for diffusing the light of the light source (3), such that the light exits the diffuser body (10) at a predefined intensity in different parts over the length of the diffuser body (10), and wherein
the light diffuser (1) further comprises a transparent sleeve surrounding the diffuser body (10), the transparent sleeve having a smooth outer surface,
**characterized in that**
the light diffusing structures (11‴) are formed as radial holes on the diffuser body (10), and **in that**
the radial holes increase in diameter and/or in frequency and/or in depth from the first end (10a) of the diffuser body (10) to the second end (10b) of the diffuser body (10).

2. The light diffuser (1) according to claim 1, wherein the predefined intensity in different parts varies between the different parts of the diffuser body (10).

3. The light diffuser (1) according to claim 1, wherein the predefined intensity in different parts is uniform over the length of the diffuser body (10).

4. The light diffuser (1) according to any of the preceding claims, wherein the first material of the diffuser body (10) comprises polymethylmethacrylate (PMMA).

5. The light diffuser (1) according to any of the preceding claims, wherein the elongated diffuser body (10) is cylindrical.

6. The light diffuser (1) according to any of the preceding claims, wherein the radial holes are through holes that extend through the diffuser body (10).

7. The light diffuser (1) according to any of the preceding claims, wherein the diffuser body (10) further comprises fillers in at least some of the radial holes.

8. The light diffuser (1) according to claim 7, wherein the fillers are colored.

9. The light diffuser (1) according to claim 8, wherein the color of the fillers varies depending on the region of the diffuser body (10) in which each filler is located.

10. The light diffuser (1) according to claim 9, wherein the color of the fillers located near first end (10a) of the diffuser body (10) differs from the color of the fillers located near second end (10b) of the diffuser body (10).

11. The light diffuser (1) according to any of the claims 7 to 10, wherein the fillers are acrylic or polycarbonate.

12. The light diffuser (1) according to any of the claims 7 to 11, wherein the fillers are detachably attached in the radial holes.

13. The light diffuser (1) according to any of the claims 7 to 11, wherein the fillers are integrally formed in the diffuser body (10).
